# EUROPEAN PATENT APPLICATION

(11) **EP 1 240 907 A1**
(43) Date of publication of application: **18.09.2002**
(21) Application number: 02005503.4
(22) Date of filing: 11.03.2002
(51) Int. Cl.: A61L 9/02, A61L 9/03, A01M 13/00

(54) **Device for diffusion of solutions manufacturing method thereof**

(30) Priority: 16.03.2001 IT MI010559
(71) Applicant: ZOBELE HOLDING S.P.A., 38100 TRENTO (IT)
(72) Inventor: Zobele, Franco, 38100 Trento (IT); Marchetti, Fabio, 38050 Povo (Trento) (IT)
(74) Representative: Petruzziello, Aldo

(57) **Abstract**

A device for diffusing solutions comprising a porous element (1) impregnated with said solution and a heating element to heat the porous element allowing evaporation of the active principles contained in the solution absorbed by the porous element (1), the porous element comprising the heating element in the form of a resistive material (2) deposited on part of the outer surface, electrical contacts (5) designed for connection to an electrical power source being provided on the layer of resistive material.

## Description

The present invention relates to a device for diffusing solutions, particularly solutions containing active principles, such as insecticide, deodorant and disinfecting substances and the like. The present invention further refers also to a method of manufacturing said device and to an electric diffuser using said device.

The systems for diffusing active principles into the air currently in use are based on heating a support containing the product to be evaporated to pre-set temperatures, according to the type of product. Said systems essentially comprise, besides the parts for containing and for connection to the power system, an element containing the substance to be evaporated and a heating element.

As far as the parts containing the product are concerned, there exist single-dose systems, such as a tablet of porous material impregnated with the active principle to be diffused, or systems provided with a refill and a porous element which conveys the liquid from the reservoir to the evaporating area. These last devices allow a number of cycles of use and thus diffusion of the product for a longer period of time.

The heating part, on the other hand, consists of a heat source, a metal layer or oxide resistance or piezoelectric element (PTC), and an element of ceramic material that ensures electrical insulation. Said heating part has a high capacity and thermal conduction and thus allows the temperature to be made uniform with respect to the part where the product to be diffused is introduced.

Heating of the element containing the product takes place either by conduction, when said element is in direct contact with the heating body, or by convection: first the air between the heating element and the porous element is heated and then the air subsequently heats the porous element.

Given the thermal characteristics of the materials making up the porous supports currently in use, possible small variations in shape or position with respect to the heating element can be reflected in different temperatures inside the porous elements and therefore develop different conditions of evaporation of the product besides possible alterations in the structure of said porous elements.

Since the amount of raw material involved in the heating and evaporation process is relatively large (heating element and element containing the product to be evaporated), the power consumption of conventional devices is about 4 watts.

Furthermore, as the heating part is in contact with some points of the supporting structure, the materials, normally plastic, forming said devices must have particular characteristics, above all as far as their thermal properties are concerned.

Another important aspect of the devices in use thus far concerns their low versatility as regards diffusion of different products. In fact, once the supply voltage has been set, the single devices always reach a well-defined operating temperature, determined by the electrical characteristics of the heat source. These operating conditions are optimised for electrical diffusion of a specific product. For different products, for example products with greater or lesser volatility, the same working conditions lead to a higher or lower evaporation speed and possibly an alteration of the product and therefore they may prove unsuitable.

The object of the present invention is to overcome the drawbacks of the prior art, by providing a diffuser device for active principles that is efficient, effective and versatile.

This object is achieved according to the invention with the characteristics listed in appended independent claim 1.

Another object of the present invention is to provide a manufacturing method for manufacturing a device for diffusion of active principles that is practical, economical and simple to carry out.

This object is achieved in accordance with the invention with the characteristics listed in appended independent claim 7.

Another object of the present invention is to provide an electric diffuser with interchangeable refills that occupies little space and allows less waste of electrical power.

This object is achieved in accordance with the invention with the characteristics listed in appended independent claim 10.

Another object of the present invention is to provide an electric diffuser with disposable refills that is practical and economical.

This object is achieved in accordance with the invention with the characteristics listed in appended independent claim 13.

Advantageous embodiments of the invention are apparent from the dependent claims.

The peculiar characteristic of the device for diffusion of active principles according to the invention is the integration on one and the same element of two essential functions for the diffusion of active principles: a product container and a heating system.

This is obtained by deposition on the surface of the porous element of a layer of material characterized by a specific resistivity per surface unit. Electrodes that can be connected to the electrical power mains are provided on said layer of material. When a fixed voltage is applied to the layer of material, an electric current passes through it and by Joule effect it produces heat that causes heating of the substrate formed by the porous element and the resulting evaporation of the product held in the container.

The device for diffusion of active principles according to the invention has various advantages.

The device according to the invention is less bulky than prior art devices. In fact the resistive layer deposited on the surface of the porous element entirely replaces the external heating element of the present electric diffusers, therefore the diffuser device can be markedly reduced in size because the part relating to the external heating element is no longer present.

The device according to the invention allows a considerable cost saving in the choice of materials for production of the container. In fact, given that the heat source is situated only on the porous element, the adjacent structure can be made of materials with less binding technical characteristics.

The device according to the invention allows a greater energy saving with respect to the devices of the prior art. In fact, the deposited surface layer of resistive material optimises heat distribution on the element containing the product and the better thermal contact between the heating element and the substrate allows working temperatures to be reached very rapidly. Consequently, the better thermal contact and optimisation in the heat distribution make it possible to operate at powers (about 2 watts) that are lower than the operating powers of prior art devices.

The device according to the invention proves extremely versatile. In fact, the heating element can be deposited on porous elements of any shape. Furthermore, since the heating element is associated with the porous element and therefore with the specific refill, the same diffusion device can be used indifferently for products with different volatility characteristics.

Further characteristics of the invention will be made clearer by the detailed description that follows, referring to purely exemplary and therefore non-limiting embodiments thereof, illustrated in the appended drawings, in which:
Figures 1-3 are diagrammatic axonometric views illustrating the various stages of production of a device for diffusion of active principles according to the invention;
Figure 4 is a diagrammatic front view of an electric diffuser according to the invention;
Figure 5 is a top plan view of a device for coupling of a refill for the diffuser of Figure 4;
Figure 6 is a side view of the coupling device of Figure 5;
Figures 7 -9 are respectively front, rear and side views of a refill for an electric diffuser according to a second embodiment of the invention;
Figure 10 is an exploded side view of an electric diffuser according to a second embodiment of the invention;
Figure 11 is a front view of the assembled electric diffuser of Figure 10, in which a closing element has been removed and the axis joining the poles of the plug is disposed parallel to a horizontal plane;
Figure 12 is a view, like Figure 11, wherein the closing element has been added and the level of the liquid contained in the refill container is shown,
Figure 13 is a front view, like Figure 11, wherein the axis joining the poles of the plug is disposed at right angles to a horizontal plane;
Figure 14 is a view, like Figure 13, in which the closing element has been added and the level of the liquid contained in the refill container is shown;
Figures 15 and 16 are respectively a front view and a rear view of a refill for an electric diffuser according to a third embodiment of the invention;
Figure 17 is a sectional view taken along the section line XVII-XVII of Figure 15;
Figure 18 is an exploded side view of an electric diffuser according the third embodiment of the invention;
Figures 19 and 20 are respectively a side view and a front view of the electric diffuser of Figure 18, assembled.

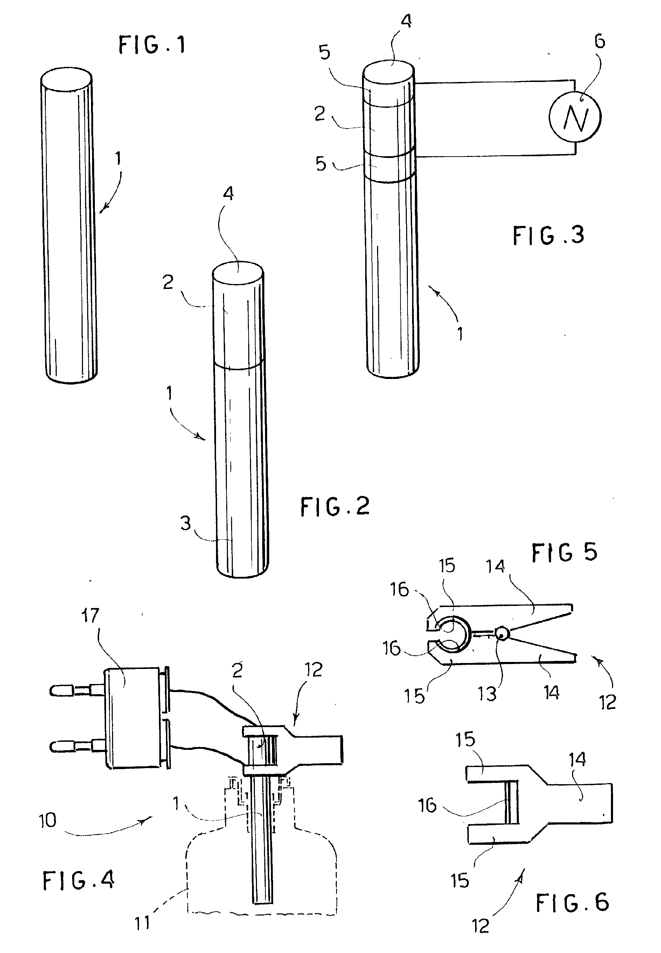

The device for diffusing active principles according to the invention is described with the aid of Figures 1-3.

Figure 1 shows a porous element having the form of a cylindrical column. The element 1 is made of a porous material able to absorb liquid solutions containing active principles.

As shown in Figure 2, a layer of resistive material 2 is deposited on the surface of the porous element 1. The resistive material 2 is characterized by a specific resistivity per surface unit. The resistive material 2 is deposited at one end of the side surface of the porous element 1, leaving free the other end 3 which is designed to be inserted in the container of the liquid with active principles.

Since the resistive material 2 is deposited only on the side surface of the porous element 1, the upper end 4 of the porous element 1 remains without the layer of resistive material 2 and acts as an evaporating surface to allow evaporation of the liquid solution containing the active principles absorbed by the porous element 1.

As shown in Figure 3, two electrical contacts 5 of conductive material are formed at the ends of the layer of resistive material 2. The electrical contacts 5 are connected by means of electrical cables to an electric power source 6 or to a plug to be inserted in a socket outlet of the electrical mains. Thus a fixed voltage is applied to the resistive layer 2 and an electric current passes through it which produces heat through a Joule effect, causing heating of the substrate formed by the porous element 1 and consequent evaporation of the liquid with active principles from the evaporating surface 4.

The resistive surface layer 2 consists of a material that is obtained by mixing two inks: one graphite-based with very low resistivity and the other with insulating characteristics. The different concentration ratios of the two components allow the resistance of the layer to be varied. To optimise the viscosity of the product and thus facilitate deposition thereof, a certain amount of diluting substance can be added to the ink mixture.

Deposition of this material can be performed by various methods: silkscreen printing, spray deposition, immersion deposition or others according to the type and shape of the porous element that is to be treated.

The shape of the deposited area is not an essential parameter for operation of the device and can be varied on a case by case basis in order to optimise heating of the porous element 1 and connection thereof to the electrical supply system, and this depends largely on the shape of said porous element. However, the deposit must not cover the entire porous element in that part of the surface thereof must be left free to allow release of the evaporated product. Thus, for example, in the case of porous elements with a cylindrical symmetry, such as the wicks used in multiple dose diffusers, the layer is deposited on the outer surface of the terminal part of the cylinder, the one not inserted in the refill, as shown in Figure 2. In the case of planar porous elements (tablets but also flat wicks), on the other hand, deposition must be carried out on one of the two surfaces of the element.

Once deposition has been performed, the system must undergo a thermal treatment that allows drying and polymerization of the resistive layer 2. The parameters of this treatment, temperature and time, depend on the type of inks that are used and the characteristics of the materials forming the porous elements. Normally these thermal elements are taken to conventional hot-air ovens. However, it is also possible to use infrared lamps and thus radiant heating.

After the thermal treatment the porous heating element 1 is ready to be impregnated with the product to be evaporated and subsequently to be inserted and used in the relative diffuser devices.

An essential characteristic of said devices must be the possibility of developing a safe electrical contact with the resistive layer 2, that remains stable over time and always in the same position, so that the dimensions of the resistive layer and thus the resistance do not change, though allowing easy insertion of a new refill or removal of a spent refill without a deterioration in the functional characteristics and performance of said device.

By way of example, Figure 4 shows an electric diffuser 10 that uses a cylindrical porous heating element 1 inserted in a multiple dose refill 11 that comprises a container with a solution containing active principles.

The electric diffuser 10 provides a coupling device 12 to allow coupling/uncoupling of the refill 11 and the electrical contact with the resistive layer 2.

As shown in Figures 5 and 6, the device 12 comprises two levers 14, defining two end jaws 15, coupled by means of a torsion spring 13. By operating the outer levers 14 the spring 13 is loaded and thus the jaws 15 of the device 12 open and this allows either insertion of a new refill 11 or release of the spent refill.

On releasing the outer levers 14, if a refill is present, the jaws 15 always grip the upper part of the porous element 1, that is to say the part on which the resistive layer 2 is deposited, in the same position. Metal blades 16, stably connected with an electric power plug 17 are positioned at a fixed distance on the ends of the coupling device 12 in which coupling with the porous element takes place. In this manner, the metal blades 16 of the coupling device 12 allow the electrical contact with the surface resistive layer 2 and connection thereof to the electrical supply system.

The coupling device 12 can also be provided with all those switch devices, possibly provided with timers that allow the electric diffuser 10 to be turned on and off without necessarily disconnecting the device from the electrical supply system.

The electric diffuser 10 has been designed with a coupling device 12 for cylindrical porous elements 1.

The electric diffuser 10 is not of the disposable type, that is to say is can be used with a large number of refills. Consequently, the coupling device 12 is designed for coupling/uncoupling of the product refill. Furthermore, the coupling device 12 must have a mechanical system which, by ensuring reproducibility of positioning of the refill, always allows a good electrical contact to be obtained between the resistive film 2 deposited on the porous element 1 and the elements of the electric diffuser that connect the electric supply system.

A second and a third embodiment of electric diffusers of the disposable type using porous elements with a deposited resistive film according to the invention will be illustrated below.

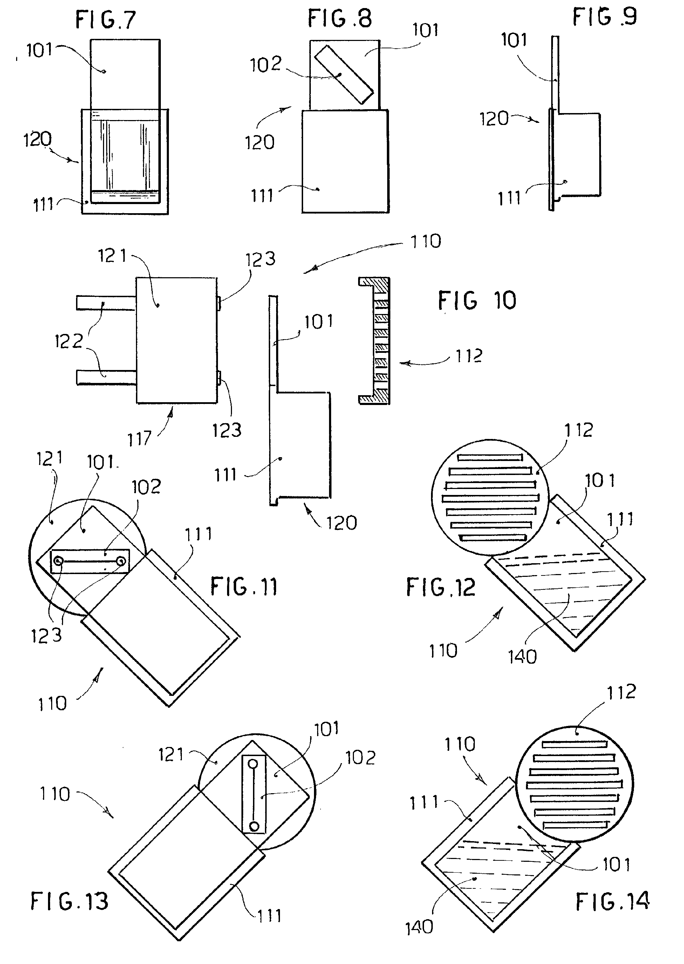

A second embodiment of the electric diffuser according to the invention is described with the aid of Figures 7-14.

Figures 7-9 show a refill denoted as a whole by reference numeral 120. For the sake of clarity, the term refill is used, even if it is destined to be incorporated in an electric diffuser of the disposable type.

The refill 120 comprises a container 111 wherein the solution with active principles is contained, a plate-shaped porous element 101 partially inserted in the container 111 and a heating element 102 deposited on the porous element 101.

The porous element 101 consists of a material such as cardboard, plastic or other porous material that allows the liquid to be conveyed by capillarity from the container 111 to the evaporation area outside the container.

The heating element 102, in the form of a thin film or resistive material, is deposited on the porous element 101, as already described. The heating element is in the form of a strip inclined 45° with respect to the axis of the porous element.

The container 111 can consist of any transparent plastic material, to allow direct visual checking of the amount of liquid present therein. The container 111 can comprise one or more parts and must be made so as to provide a completely tight seal, also in the region of the porous element 101, to prevent the liquid contained from escaping or the porous element from being removed, independently of the orientation 120 in which the refill is positioned.

Figure 10 is an exploded view of an electric diffuser 110 according to a second embodiment. The electric diffuser 110 comprises the refill 120, an electric plug 117 and a closing element 112. The electric plug 117 comprises a body 121, two poles 122 for connection to the electric mains and two electrical contacts 123 destined to come into contact with the resistive element 102 deposited on the porous element 101.

The closing element 122 is responsible for blocking the plug 117 so that the electrical contacts 123 press on the resistive element 102. The closing element is made of insulating material to ensure adequate electrical insulation of the biased parts and has a grille or slotted configuration to allow the products evaporated during operation of the electric diffuser 110 to escape into the environment. Since the electric diffuser 110 is disposable, assembly thereof is done so as to avoid further opening.

Figures 11 and 13 show the electric diffuser 110, positioned respectively in an electric socket in which the axis 130 joining the holes that receive the pins 122 of the plug 117 is parallel to a horizontal plane and in an electric socket in which the axis 130 is at right angles to a horizontal plane. Thanks to the fact that the resistive film 102 is positioned at 45° with respect to the axis of the porous element 101, as shown in Figures 12 and 14, in both positions the porous element 101 has a part inside the container 111 that ensures contact with the liquid 140 contained therein, until it is all used, without the need to provide the electric diffuser 110 with a rotating plug.

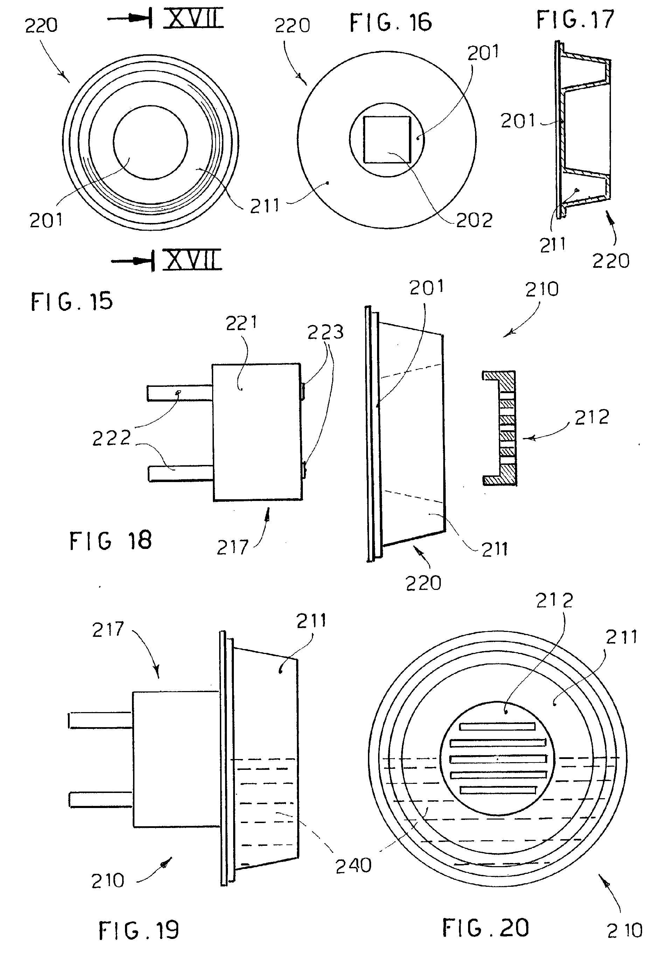

A third embodiment of the electric diffuser according to the invention is described with the aid of Figures 15-20.

Figures 15-20 show a refill denoted as a whole with reference numeral 220. The refill 220 comprises a toroidal or doughnut-shaped container 211 wherein the solution with the active principles is contained, a porous element 201, in the form of a discoid blade, partially inserted in the container 211 and a heating element 202 deposited in the central area of the porous element 201 which is not covered by the container 211.

The porous element 201 is made of material such as cardboard, plastic or another porous material that allows the liquid to be conveyed by capillarity from the container 211 to the central area of the porous element 201 which represents the evaporating area.

The container 211 can be formed of any transparent plastic material to allow direct visual checking of the amount of liquid present therein. The container 211 can comprise one or more parts and must be made so as to ensure a tight seal, also in the area in contact with the porous element 201, to prevent the liquid contained therein from escaping or the porous element 201 from being removed, independently of the orientation in which the refill 220 is positioned.

Figure 18 is an exploded view of an electric diffuser 210 according to the third embodiment. The electric diffuser 210 comprises the refill 220, an electric plug 217 and a closing element 212 essentially similar to those already described in the second embodiment. The electric plug 217 comprises a body 221, two pins 222 for connection to the electric mains and two electrical contacts 223 destined to come into contact with the resistive element 202 deposited in the central part of the rear surface of the porous element 201.

As in the second embodiment, the closing element 212 is responsible for blocking the plug 217 so that the electrical contacts 223 press on the resistive element 202. The closing element is made of insulating material to ensure adequate electrical insulation of the live parts and has a grill-type or slotted configuration to allow the evaporated products to escape into the environment during operation of the electric diffuser 210. Assembly of the electric diffuser 210 is done so as to avoid further opening.

Figures 19 and 20 show the assembled electric diffuser 210, in which the container 211 is transparent to show the level of the liquid 240 contained therein. The cylindrical symmetry of the electric diffuser 210 allows it to be used independently of the wall-mounted electric socket outlets in which it is inserted, without the need to provide a rotating plug. In fact a part of the porous element 201 always remains in contact with the liquid 240 inside the container 211 until it is all used.

Numerous variations and modifications of detail within the reach of a person skilled in the art can be made to the present embodiments of the invention without departing from the scope of the invention expressed by the appended claims.

## Claims

1. A device for diffusion of solutions containing active principles comprising a porous element (1) that is impregnated with said solution and a heating element to heat said porous element allowing evaporation of said active principles contained in the solution absorbed by said porous element (1), **characterised in that** said porous element (1) comprises said heating element in the form of a layer of resistive material (2) deposited on part of the outer surface.

2. A device according to claim 1, **characterised in that** on said layer of resistive material (2) there are electrical contacts (5) destined to be connected to an electric power source.

3. A device according to claim 1 or 2, **characterized in that** said layer of resistive material (2) comprises a mixture of two inks, one graphite-based with low resistivity and the other with insulating characteristics.

4. A device according to any one of the preceding claims, **characterized in that** a part (4) of the surface of said porous element (1) is devoid of said layer of resistive material (2) to allow evaporation of the active principles.

5. A device according to any one of the preceding claims, **characterized in that** said porous element (1) is substantially cylindrical in shape and said layer of resistive material (2) is deposited on the side surface at one end of said porous element (1).

6. A device according to any one of claims 1 to 3, **characterized in that** said porous element (1) is substantially in the form of a plate and said layer of resistive material (2) is deposited on one face of said porous element (1).

7. A manufacturing method for a device for diffusing solutions of active principles comprising the following steps:
- production of a porous element (1) able to absorb a solution containing active principles;
- deposition of a layer of resistive material (2) on part of the surface of said porous element (1);
- heat treatment of said porous element (1) to allow drying and polymerisation of said layer of resistive material (2).

8. A method according to claim 7, **characterized in that** said step of deposition of a layer of resistive material (2) comprises silkscreen printing, spray deposition, or immersion deposition.

9. A method according to claim 7 or 8, **characterized in that** said heat treatment step comprises treatment in a conventional hot-air oven or radiant heating by means of infrared lamps.

10. An electric diffuser (10) for diffusion of solutions containing active principles comprising:
- a refill (11) consisting of a container inside which a solution containing said active principles and a diffusion device is contained comprising a porous element (1) impregnated with said solution having a part of the surface on which a layer of resistive material (2) is deposited, and
- a coupling device (12) for coupling/uncoupling of said refill (11) and for the electrical contact with said layer of resistive material (2) of said porous element, said coupling device (12) being connected electrically to an electric power plug (17).

11. An electric diffuser (10) according to claim 10, **characterized in that** said coupling device (12) comprises two levers coupled by means of a torsion spring, said two levers generating two jaws (15) to clamp said porous element (1) on the layer of resistive material (2).

12. An electric diffuser (10) according to claim 10 or 11, **characterized in that** said coupling device (12) comprises two metal blades (16) electrically connected to said electric power plug (17), said blades (16) coming into contact with said layer of resistive material (2) deposited on the porous element (1).

13. An electric diffuser (110; 210) for diffusion of solutions containing active principles comprising:
- a refill (120; 220) comprising:
- a container (111; 211) wherein a solution containing said active principles is contained,
- a porous element (101; 201) impregnated with said active principles, and
- a layer of resistive material (102; 202) deposited on a part of the surface of said porous element,
- an electric plug (117; 217) for connection to an electric socket outlet, comprising electrical contacts (123; 223) able to go into contact with said resistive layer (102; 202) deposited on the porous element, and
- a coupling device (112) able to couple said electric plug (117; 217) to said refill (120; 220) so that said electrical contacts (123, 223) of the electric plug go into contact with said resistive layer (102; 202) deposited on the porous element.

14. An electric diffuser (110) according to claim 13, **characterized in that** said porous element (101) is in the form of a plate partially inserted into said container (111) and said resistive layer (102) is a strip disposed on the part of the porous element outside the container, with an inclination of about 45° with respect to the axis of said porous element.

15. An electric diffuser (210) according to claim 13, **characterized in that** said porous element (201) is in the form of a discoid plate inserted in said toroidal container (211) so as to leave the central part of said resistive layer uncovered and said resistive layer (202) is a strip disposed on the central part of the porous element.

16. An electric diffuser (110; 210) according to any one of claims 13 to 15, **characterized in that** said coupling device (112) is of insulating material to ensure electrical insulation and has a shape having holes or slots to allow diffusion of the active principles.
